(19) Europäisches Patentamt
European Patent Office
Office européen des brevets

(11) Publication number: **0 422 693 B1**

# EUROPEAN PATENT SPECIFICATION

(45) Date of publication of patent specification: **21.06.95**

(21) Application number: **90123938.4**

(22) Date of filing: **06.01.86**

(60) Publication number of the earlier application in accordance with Art.76 EPC: **0 211 851**

(51) Int. Cl.⁶: **C08L 75/04**, C08L 71/00, C08L 67/00, A61L 29/00, A61K 9/22, A63H 3/44, B29C 61/00, //(C08L75/04, 71:00),(C08L71/00,75:04), (C08L71/00,67:00),(C08L67/00, 71:00)

(54) **Method for making an article with shape-memory properties and some of the thus obtained articles.**

(30) Priority: **04.01.85 US 688793**

(43) Date of publication of application:
**17.04.91 Bulletin 91/16**

(45) Publication of the grant of the patent:
**21.06.95 Bulletin 95/25**

(84) Designated Contracting States:
**AT BE CH DE FR GB IT LI LU NL SE**

(56) References cited:
EP-A- 0 087 329     EP-A- 0 129 396
DE-A- 1 704 160     FR-A- 2 546 170
US-A- 3 384 679     US-A- 3 619 455
US-A- 4 053 548     US-A- 4 193 899
US-A- 4 473 671

(73) Proprietor: **THORATEC LABORATORIES CORPORATION**
**2023 Eighth Street**
**Berkeley, CA 94710 (US)**

(72) Inventor: **Ward, Robert S.**
**760 Tanglewood Lane**
**Lafayette,**
**California 94549 (US)**
Inventor: **Riffle, Judy S.**
**6956 Saroni Drive**
**Oakland,**
**California 94611 (US)**

(74) Representative: **Baverstock, Michael George Douglas et al**
**BOULT, WADE & TENNANT**
**27 Furnival Street**
**London, EC4A 1PO (GB)**

Alfred Rudin "The Elements of polymer science and engineering" 1982, Academic Press , New York; pages 394-395, 398-399.

D.W.Van Krevelen "Properties of Polymers" 1990 Elsevier, Amsterdam; pages 286, 303, 374-375, 380, 382.

## Description

Generally, the present invention relates to a method for making an article with shape memory properties using a composition that softens at a predetermined or selected temperature between approximately room temperature and approximately 62°C (about 25°C above body temperature) but maintains good mechanical integrity well above that softening point. The present invention also relates to biomedical devices and materials and articles with shape-memory properties made by this process.

The used composition is ideally suited for biomedical uses and is especially suited for catheters. Because catheters are inserted into the body, the ideal catheter should have a high flexural modulus or stiffness similar to that of a hypodermic needle. This is particularly true for intravenous catheters which are inserted through the skin for a short distance and then inserted directly into a blood vessel. Since ease of insertion is a property that is most important in initially placing the catheter, intravenous catheters have always been made from high flexural modulus materials. Once the catheter is in place inside the vein, the stiff property or high flexural modulus is a distinct disadvantage. High flexural modulus can result in irritation of the inner surface of the blood vessel by the tip of the catheter. As a result of the inflammatory irritation to the inside of the blood vessel, it is very common for these catheters to produce blood clots and become infected.

A very soft, rubber-like material would have much less tendency to irritate the inside of the blood vessel and would result in reduced morbidity and increased life of the catheter (the length of time before the catheter has to be replaced or moved to a different site). However, a flexible catheter would lack ease of insertion. Thus, there is a need for a catheter which is both easy to insert and does not irritate the inside of the blood vessel. The ideal catheter composition would have a very high modulus or stiffness upon insertion and would soften once inside the blood vessel.

It is an object of the present invention to provide a process using compositions that soften at a predetermined or selected temperature between approximately room temperature and approximately 62°C (about 25°C above body temperature) but maintain good mechanical integrity above that softening point.

It is another object of the present invention to provide articles with shape memory properties that soften at predetermined temperature between 20°C and 62°C.

US-A-4053548 described a process for making shape memory articles comprising the steps of:

a) forming a thermoplastic composition comprising a block copolymer A-B-A in a first shape at a temperature above both softening points of A and B

b) reforming to a second shape the formed thermoplastic of step (a) at a temperature between that of the softening points of the A and B blocks; and

c) cooling the reformed thermoplastic to a temperature below both softening points while retaining said new shape.

According to the present invention there is provided a method for making an article with shape-memory properties comprising the steps of heating a composition comprising at least one block copolymer and a polymer, said polymer being selected from polyvinylchloride, styreneacrylonitrile copolymers, polyhydroxyethers, polycarbonates, polyesters, polyethers, nitrocellulose and polystyrene and being thermodynamically compatible with at least one block of said block copolymer and having at least one thermal transition temperature, said block copolymer being selected from block copolymers having a polyester block, block copolymers having a polyether block, and polyurethane/ureas and having a lower thermal transition temperature and an upper thermal transition temperature, said composition characterized by at least two thermal transition temperatures, one of said transition temperatures being a predetermined lower thermal transition temperature in the range of 20°C to 62°C, said predetermined composition lower thermal transition temperature being between said block copolymer lower thermal transition temperature and said polymer at least one thermal transition temperature, the other of said composition thermal transition temperatures being a composition upper thermal transition temperature above 62°C, said composition further characterized by the physical properties of a polymer selected from rigid, glassy polymers and rigid, crystalline polymers, below said composition predetermined lower thermal transition temperature and the physical properties of a flexible, elastomeric polymer at and above said composition predetermined lower thermal transition temperature but below said composition upper thermal transition temperature, to a temperature above said composition upper thermal transition temperature, orienting said composition under stress into a first configuration, cooling said composition to below said composition lower thermal transition temperature while said composition is in said first configuration, and cold forming said composition into a second configuration.

Specially preferred is that the block copolymer comprises a polyester-polyether.

According to a variation said block copolymer comprises polyester urethane and said polymer comprises phenoxy resin or the block copolymer comprises a polyester-polyether and said polymer comprises phenoxy resin.

The used composition should comprise 30 to 70 weight percent of said phenoxy resin and 70 to 30 weight percent of said polyester-ether.

It is still preferred that the transition temperature is at body temperature and said first configuration is a catheter or a drug-release device or a thermal indicator.

The predetermined glass transition temperature should be in the range of from 10°C above body temperature to 62°C.

Finally, the object made by the method of the invention may be a toy with shape-memory properties as obtained by the above-mentioned process.

The composition used in the method of the present invention comprises at least one block copolymer and a polymer that is thermodynamically compatible with at least one block of said block copolymer. Other blocks or segments of the block copolymer base are thermodynamically incompatible with the second polymer. The second polymer has at least one thermal transition temperature, (either a Tg or Tm) and the block copolymer base has at least two thermal transition temperatures.

The block copolymer or segmented copolymer is the base polymer of the composition. The second polymer is blended together with the base polymer. The compatible blocks of the base polymer and the second polymer form a miscible blend with a transition temperature between the thermal transition temperature of the compatible block of the base polymer and the thermal transition temperature of the second polymer. The transition temperature of the miscible blend may be varied by varying the relative amounts of the two components. The blocks or segments of the base polymer which are incompatible with the second polymer should have a Tg or Tm higher than the temperature range to he encountered in the end use of the composition of the present invention. The incompatible block of the base polymer will have a Tg or Tm in the composition very close or equal to the Tg or Tm it had in the unblended base polymer.

At temperatures above the predetermined transition temperature of the used composition, the blend will be analogous to a thermoplastic elastomer with a soft block transition temperature that may be varied over a wide range of temperatures. The temperature range over which the transition takes place will narrow if the two miscible components are highly compatible, producing an abrupt change in the mechanical, thermal, transport, electrical, and other properties of the blend as the temperature is raised or lowered through the transition temperature.

The properties of the blend at temperatures above and below the predetermined transition temperature can be varied considerably by varying the composition of the base polymer. Greater concentration of the miscible or compatible block in the base polymer will result in a greater proportion of the blend subsequently possessing the predetermined transition temperature of the composition. For instance, in a 60/40 blend of a polycaprolactone(PCL)/diisocyanatodiphenylmethane(MDI)/butanediol(BD) polyurethane base polymer and phenoxy resin, the soft block PCL concentration of the base polymer may be varied. At very low PCL concentration, the blend will be very rigid below the predetermined transition temperature and will soften little when heated above the predetermined transition temperature. At high PCL concentration in the base polymer, the blend will soften dramatically above the predetermined transition temperature.

Similarly, the incompatible blocks or segments of the base polymer may be chosen to vary the properties of the blend. A hard block composed of hexamethylenediisocyanate and butanediol would have a lower transition temperature than a rigid block of diisocyanatodiphenylmethane and butanediol within the range of end use temperatures.

The compatible block or blocks in the base polymer and the second polymer have special requirements relative to each other. In addition to being thermodynamically compatible as determined by a single transition temperature for the blend, the block copolymer (base polymer) and second polymer are selected such that the lower transition temperature (Tg or Tm) of the block copolymer is different than the transition temperature (Tg or Tm) of the second polymer, one of the transition temperatures being higher and one lower than the desired or predetermined transition temperature of the composition. Generally, the compatible block of the base polymer will have the lower Tg or Tm and the second polymer will have the higher Tg or Tm. As the second polymer is added to the base polymer, the Tg or Tm of the soft black or segment of the base polymer increases. However, it is also possible to use a higher Tg or Tm base polymer with two hard blocks only one of which is compatible with the lower Tg or Tm polymer. As the lower Tg or Tm second polymer is added to the base polymer, the Tg or Tm of the compatible block of the base polymer decreases. In both cases, the relative amount of the second polymer will determine the final Tg or Tm of the compatible block of the base polymer and accordingly, the predetermined thermal transition temperature of the blend.

The composition used in the method of the present invention is further characterized by the physical properties of a rigid, glassy, semi-crystalline, or crystalline polymer below the predetermined transition temperature and the physical properties of a soft, rubbery polymer at and above the predetermined transition temperature, but below the upper transition temperature of the composition. The physical or mechanical properties of glassy or crystalline polymers and rubbery polymers are described in "Mechanical Properties of Polymers and Composites," L.E. Nielsen, Dekker, N.Y. (1974), and "Properties of Polymers, Their Estimation and Correlation with Chemical Structure," D. W. Van Krevelen, Elsevier, N.Y. (1976), and are well known to those skilled in the art.

For biomedical uses, a preferred embodiment of the composition used in the present invention has a predetermined glass transition temperature between body temperature and 62°C. For certain biomedical applications, such as making catheters, drug release devices, or thermal indicators, a preferred embodiment of the composition has a glass transition temperature at or near body temperature.

For biomedical applications, a preferred composition used in the present invention comprises at least one block copolymer and a second polymer thermodynamically compatible with at least one block of said block copolymer, wherein the block copolymer is characterized by at least two thermal transition temperatures, one being below body temperature and the other being well above body temperature, preferably above 62°C. The second polymer has at least one transition temperature (either a Tg or Tm) above body temperature.

The block copolymer (base polymer) is selected from the group consisting of polyester urethanes, polyether urethanes, polyurethane/ureas, polyether polyamides, polyether polyesters, and any other block or segmented copolymers containing at least one polyether or polyester block or segment. One preferred block copolymer (base polymer) is a polyester urethane. Polyester urethanes have glass transition temperatures of -50°C to -10°C and 100°C to 150°C. Another preferred block copolymer is a polyether urethane. Polyether urethanes have glass transition temperatures of -85°C to -40°C and 100°C to 150°C.

The second polymer is selected from the group consisting of polyvinylchloride, styrene/acrylonitrile copolymers, polyhydroxyethers, polycarbonates, polyesters, polyethers, nitrocellulose, and polystyrene. A preferred second polymer is the polyhydroxyether of bisphenol-A and epichlorohydrin (phenoxy resin). The phenoxy resin has a glass transition temperature of 98°C.

The two polymers are then blended in a ratio so that the predetermined lower transition temperature for the composition will be at or near body temperature or slightly above body temperature if hydration lowers this glass transition temperature further.

For making catheters or other biomedical applications, the composition used in the method of the invention may also contain a minor amount of a surface modifying additive. The surface modifying additive reduces the coefficient of friction and increases blood compatibility of the composition. In addition, radiopaque fillers may be used in the composition to render the catheters visible by fluoroscopy. Surface modifying additives may be selected from the group consisting of block/segmented multipolymers containing polysiloxanes or polyfluorocarbons as one block. Preferred surface modifiers are selected from the group consisting of polysiloxane/polyester block copolymers and polysiloxane/polyurethane copolymers. A more preferred surface modifier is a polysiloxane/polyester block copolymer. Radiopaque fillers may be selected from the group consisting of barium sulphate, bismuth sub-carbonate, bismuth oxychloride, tantalum and other non-corrosive metals. A preferred radiopaque filler is barium sulphate.

For use as a catheter, a preferred embodiment of the composition used in the method of the invention has a glass transition temperature at or near body temperature, such that when the composition is at room temperature, it is below its glass transition temperature and is stiff and rigid, having the physical properties of a glassy or crystalline polymer. Accordingly, a catheter made by the method of the invention will be easy to insert through the skin and not the blood vessel. As the composition warms to body temperature, it will go through the transition and its flexural modulus will be decreased by about one to three orders of magnitude, causing the composition to soften considerably. Thus, while in the blood vessel the catheter will have less tendency to irritate the inside of the blood vessel and thus, less tendency to cause blood clots and become infected.

Because most intravenous catheters have thin walls, they have a tendency to heat up very quickly when exposed to a new temperature. As a result, the catheter can actually begin to soften as it is being inserted into the body, making insertion difficult. Therefore, a more preferred embodiment of the composition used in the method of the present invention has a predetermined lower thermal transition temperature slightly above body temperature. When the catheter is inserted into the body, the composition absorbs water from the blood and becomes plasticized; its predetermined lower transition temperature is lowered from slightly above body temperature to just below body temperature. The composition then goes through its transition to become a rubbery, flexible solid.

For other biomedical applications, a preferred embodiment of the composition used according to the method of the present invention will have a predetermined lower transition temperature above body temperature but below 62°C and an upper transition temperature above 62°C, such that the composition has the physical properties of a rigid, glassy or crystalline polymer at about body temperature and the physical properties of a flexible, rubbery polymer at 10°C above body temperature to just below the upper transition temperature.

Thus, for instance, a catheter with shape-memory properties could be made by the method of the present invention. The composition could be formed into a catheter in one configuration above its upper transition temperature then cooled to below a lower transition temperature at or near body temperature and formed into a second configuration (e.g., a straight configuration). Upon insertion into the body, the catheter would warm to body temperature (its lower glass transition temperature) and reassume its first configuration.

Another use of the present invention would be for the production of toys with shape-memory properties. The composition could be oriented under stress into one toy shape above the composition's upper transition temperature. When cooled below the predetermined lower transition temperature, the toy could be reshaped by children. Then, the toy could be warmed to above its predetermined lower transition temperature, thus reassuming its original shape.

The various blocks or segments of the block copolymer are selected such that the physical properties of the composition are that of a rigid, glassy or crystalline polymer below the predetermined lower transition temperature of the composition and that of a flexible, rubbery polymer at and above the lower transition temperature, but below the upper transition temperature.

The block copolymer selected for use in the composition of the present invention can be made by a method described in: 1. J. A. Moore, ed., Macromolecular Syntheses, Collective Vol. 1, John Wiley and Sons, N.Y., (1977), p. 79,381. 2. J. H. Saunders, and K.C. Frisch, eds., Polyurethanes, Chemistry and Technology, Vol. 1, Chemistry, Interscience Div. of John Wiley and Sons, N.Y., (1962). 3. W. R. Sorenson and T. W. Campbell, Preparative Methods in Polymer Chemistry, Interscience Div. of John Wiley and Sons, N.Y., (1961).

The method comprises the steps of selecting the desired transition temperature for the composition, selecting or making a block copolymer such that said block copolymer has at least two thermal transition temperatures. The lower transition temperature of the block copolymer should be either above or below the predetermined or selected lower transition temperature of the composition. A second polymer that is thermodynamically compatible with at least one block of the block copolymer and that has at least one thermal transition temperature (either a Tg or Tm) that is either above or below the predetermined transition temperature of the composition is selected or prepared, then blended together with the block copolymer such that the lower thermal transition temperature of the block copolymer and the thermal transition temperature of the compatible second polymer combine to form the predetermined lower transition temperature of the composition.

For biomedical uses, a surface modifier and/or a radiopaque filler can be blended together with the base polymer and thermodynamically compatible second polymer.

Biomedical devices or materials such as cathethers, drug release devices or thermal indicators that soften at a predetermined or selected temperature between body temperature and 62°C can be made by the method of the invention. In most applications, the predetermined softening temperature will be at or near body temperature.

The following table indicates the decreasing flexural modulus of a preferred composition used in the present invention for biomedical uses when it comprises various ratios of phenoxy resin and polyester urethane:

| Phenoxy Resin/Polyester Urethane Wt/Wt | Flexural Modulus [psi] | | |
|---|---|---|---|
| | 23°C Dry | 37°C Dry | 37°C Hydrated |
| 60/40 | 186400 | 159000 | 6729 |
| 50/50 | 160400 | 8950 | 602 |
| 40/60 | 93300 | 2867 | 1186 |

The flexural modulus data in the column designated "37°C Hydrated" was obtained by measuring the composition's flexural modulus after one hour of hydration in 37°C distilled water. The flexural modulus data in the two columns designated "Dry" was obtained by measuring the composition in its dry state at the designated temperatures. The following examples describe the tests from which this data was obtained and the preferred compositions for use in the method of the present invention.

Example 1

A composition comprising 60 weight percent of a resin comprised of the polyhydroxyether derived from bisphenol-A and epichlorohydrin (phenoxy resin) and 40 weight percent of a commercial polyester urethane (Estane 5707 produced by B.F. Goodrich), in the form of compression molded sheets, was measured for flexural modulus in a thermal mechanical analyzer at 23°C, 37°C and at 37°C after an hour of hydration in distilled water. The flexural modulus of the composition at 23°C was 186,400. The flexural modulus at 37°C was 159,000 and the flexural modulus after one hour of hydration in 37°C distilled water was 6,729.

Example 2

A composition comprising 50 weight percent of the phenoxy resin described in Example 1 and 50 weight percent of the polyester urethane described in Example 1, in the form of compression molded sheets, was measured for flexural modulus in a thermal mechanical analyzer. At 23°C, the composition had a flexural modulus of 160,400. At 37°C, the composition had a flexural modulus of 8,950 and after one hour of hydration in 37°C distilled water, the composition had a flexural modulus of 602.

Transition temperatures of each blend component separately and of the final composition were measured by differential scanning calorimetry (DSC) as follows:

```
Phenoxy resin:                              Tg = 91°C
Polyester urethane:                         Tg = -19°C
Blend (50% phenoxy resin/50% Estane 5707):  Tg = 39°C
```

Additionally, 40 weight percent of the phenoxy resin, 40 weight percent of the polyester urethane, and 20 weight percent of a polysiloxane-polycaprolactone block copolymer surface modifier (36 weight percent polysiloxane) were melt blended. The transition near body temperature of that composition as measured by DSC was Tg = 27°C.

Example 3

A composition comprising 40 weight percent of the phenoxy resin described in Example 1 and 60 weight percent of the polyester urethane described in Example 1, in the form of compression molded sheets, was measured for flexural modulus in a thermal mechanical analyzer. At 23°C, the composition had a flexural modulus of 93,300. At 37°C, the composition had a flexural modulus of 2,867 and after one hour of hydration in 37°C distilled water, the composition had a flexural modulus of 1186.

Example 4

A composition comprising 50 weight percent of the phenoxy resin described in Example 1 and 50 weight percent of a commercial polyether-based polyurethane (Estane 5714 produced by B.F. Goodrich) were melt blended and the transition temperatures were measured by differential scanning calorimetry (DSC) as follows:

```
Phenoxy resin:                                 Tg = 91°C
Polyether-based polyurethane:                  Tg = -48°C
Blend (50% phenoxy resin/50% polyurethane):    Tg = 22°C
```

Additionally, 40 weight percent of the phenoxy resin, 40 weight percent of the polyether-based polyurethane, and 20 weight percent of a polydimethylsioloxane-polycaprolactone block copolymer surface modifier (36 weight percent polysiloxane) were melt blended. The transition temperature occurring around

7

but below body temperature as measured by DSC was Tg = 5°C. This result indicates that the selected surface modifier is thermodynamically compatible with the base polymer and the second polymer.

Less surface modifier should be used to achieve a final Tg near 22°C. Typically for biomedical applications, about 0.5 weight percent of a surface modifier would be used.

## Claims

1. A method for making an article with shape-memory properties comprising the steps of heating a composition comprising at least one block copolymer and a polymer, said polymer being selected from polyvinylchloride, styreneacrylonitrile copolymers, polyhydroxyethers, polycarbonates, polyesters, polyethers, nitrocellulose and polystyrene and being thermodynamically compatible with at least one block of said block copolymer and having at least one thermal transition temperature, said block copolymer being selected from block copolymers having a polyester block, block copolymers having a polyether block, and polyurethane/ureas and having a lower thermal transition temperature and an upper thermal transition temperature, said composition characterized by at least two thermal transition temperatures, one of said transition temperatures being a predetermined lower thermal transition temperature in the range of 20°C to 62°C, said predetermined composition lower thermal transition temperature being between said block copolymer lower thermal transition temperature and said polymer at least one thermal transition temperature, the other of said composition thermal transition temperatures being a composition upper thermal transition temperature above 62°C, said composition further characterized by the physical properties of a polymer selected from rigid, glassy polymers and rigid, crystalline polymers, below said composition predetermined lower thermal transition temperature and the physical properties of a flexible, elastomeric polymer at and above said composition predetermined lower thermal transition temperature but below said composition upper thermal transition temperature, to a temperature above said composition upper thermal transition temperature, orienting said composition under stress into a first configuration, cooling said composition to below said composition lower thermal transition temperature while said composition is in said first configuration, and cold forming said composition into a second configuration.

2. A method as claimed in claim 1 wherein said block copolymer comprises polyester- polyether.

3. A method as claimed in claim 1 wherein said block copolymer comprises polyester urethane and said polymer comprises phenoxy resin.

4. A method as claimed in claim 1 wherein said block copolymer comprises polyester- polyether and said polymer comprises phenoxy resin.

5. A method as claimed in any one of the preceding claims wherein said predetermined transition temperature is at body temperature and said first configuration is a catheter.

6. A method as claimed in any one of claims 1 to 4 wherein said predetermined transition temperature is at body temperature and said article is a drug-release device.

7. A method as claimed in any one of claims 1 to 4 wherein said predetermined transition temperature is at body temperature and said article is a thermal indicator.

8. A method as claimed in any one of the preceding claims wherein said predetermined glass transition temperature is in the range of from 10°C above body temperature to 62°C.

9. A method as claimed in claim 1 wherein the article is a toy.

## Patentansprüche

1. Verfahren zur Herstellung eines Erzeugnisses mit Gedächtniseigenschaften, das die Stufen der Erwärmung eines Gemisches, das wenigstens ein Blockcopolymer und ein Polymer umfaßt, wobei letzteres aus der Gruppe Polyvinylchlorid, Styrolacrylnitrilcopolymeren, Polyhydroxyethern, Polycarbonaten, Polyestern, Polyethern, Nitrocellulose und Polystyrol ausgewählt wird, thermodynamisch mit wenigstens einem Block des Blockcopolymers verträglich ist und wenigstens eine Wärmeübergangstemperatur aufweist, und das Blockcopolymer unter Blockcopolymeren mit einem Polyesterblock, Blockcopolymeren mit einem Polyetherblock und Polyurethan/Harnstoffen ausgewählt wird und eine untere und eine obere Wärmeübergangstemperatur aufweist, wobei das Gemisch dadurch gekennzeichnet ist, daß es wenigstens zwei Wärmeübergangstemperaturen aufweist, von denen die eine eine vorgegebene tiefere Wärmeübergangstemperatur im Bereich zwischen 20 und 62°C ist, wobei diese vorgegebene tiefere Wärmeübergangstemperatur des Gemisches zwischen der tieferen Wärmeübergangstemperatur des Blockcopolymers und wenigstens einer Wärmeübergangstemperatur des Polymers liegt, und die andere Wärmeübergangstemperatur des Gemisches eine obere Wärmeübergangstemperatur von über 62°C ist, und daß es außerdem unterhalb der vorgegebenen tieferen Wärmeübergangstemperatur des Gemisches die physikalischen Eigenschaften eines Polymers, ausgewählt unter harten glasartigen sowie harten kristallinen Polymeren, und bei der vorgegebenen tieferen Wärmeübergangstemperatur des Gemisches und darüber, jedoch unterhalb der oberen Wärmeübergangstemperatur des Gemisches die physikalischen Eigenschaften eines flexiblen elastomeren Polymers aufweist, auf eine Temperatur über der oberen Wärmeübergangstemperatur des Gemisches, Ausrichtung des Gemisches unter Druck zu einer ersten Konfiguration, Abkühlung des Gemisches auf eine Temperatur unterhalb der tieferen Wärmeübergangstemperatur des Gemisches, während das Gemisch in der ersten Konfiguration vorliegt, und Kaltverformung des Gemisches zu einer zweiten Konfiguration umfaßt.

2. Verfahren nach Anspruch 1, worin das Blockcopolymer Polyesterpolyether umfaßt.

3. Verfahren nach Anspruch 1, worin das Blockcopolymer Polyesteturethan und das Polymer Phenoxyharz umfaßt.

4. Verfahren nach Anspruch 1, worin das Blockcopolymer Polyesterpolyether und das Polymer Phenoxyharz umfaßt.

5. Verfahren nach einem der vorhergehenden Ansprüche, worin die vorgegebene Übergangstemperatur der Körpertemperatur entspricht und die erste Konfiguration ein Katheter ist.

6. Verfahren nach einem der Ansprüche 1 bis 4, worin die vorgegebene Übergangstemperatur der Körpertemperatur entspricht und das Erzeugnis eine Vorrichtung zur Arzneimittelfreisetzung ist.

7. Verfahren nach einem der Ansprüche 1 bis 4, worin die vorgegebene Übergangstemperatur der Körpertemperatur entspricht und das Erzeugnis ein Wärmeindikator ist.

8. Verfahren nach einem der vorhergehenden Ansprüche, worin die vorgegebene Einfriertemperatur in einem Bereich von 10°C über Körpertemperatur bis 62°C liegt.

9. Verfahren nach Anspruch 1, worin das Erzeugnis ein Spielzeug ist.

**Revendications**

1. Procédé de production d'un article ayant des propriétés de mémoire de forme, comprenant les étapes consistant à chauffer une composition comprenant au moins un copolymère séquencé et un polymère, ledit polymère étant choisi entre un polymère de chlorure de vinyle, des copolymères styrène-acrylonitrile, des polyhydroxyéthers, des polycarbonates, des polyesters, des polyéthers, la nitrocellulose et le polystyrène et étant compatible du point de vue thermodynamique avec au moins un bloc dudit copolymère séquencé et ayant au moins une température de transition thermique, ledit copolymère séquencé étant choisi entre des copolymères séquencés possédant un bloc polyester, des copolymères séquencés possédant un bloc polyéther, et des polyuréthannes/urées et ayant une température de transition thermique inférieure et une température de transition thermique supérieure, ladite composition étant caractérisée par au moins deux températures de transition thermique, une desdites températures de transition étant une température de transition thermique inférieure prédéterminée comprise dans l'intervalle de 20°C à 62°C, ladite température de transition thermique inférieure prédéterminée de la composition étant située entre la température de transition thermique inférieure dudit copolymère séquencé et au moins une température de transition thermique dudit polymère, l'autre desdites températures de transition thermique de la composition étant une température de transition thermique supérieure de la composition située au-dessus de 62°C, ladite composition étant caractérisée en outre par les propriétés physiques d'un polymère choisi entre des polymères vitreux rigides et des polymères cristallins rigides, au-dessous de ladite température de transition thermique inférieure prédéterminée de la composition, et les propriétés physiques d'un polymère élastomère flexible a une température égale ou supérieure à ladite température de transition thermique inférieure prédéterminée de la composition mais inférieure à ladite température de transition thermique supérieure de la composition, à une température supérieure à ladite température de transition thermique supérieure de la composition, à orienter ladite composition sous tension suivant une première configuration, à refroidir ladite composition à une température inférieure à ladite température de transition thermique inférieure de la composition tandis que ladite composition est sous ladite première configuration, et à façonner à froid ladite composition suivant une seconde configuration.

2. Procédé suivant la revendication 1, dans lequel le copolymère séquencé consiste en un polyester-polyéther.

3. Procédé suivant la revendication 1, dans lequel le copolymère séquencé consiste en un polyesteruréthanne et le polymère consiste en une résine phénoxy.

4. Procédé suivant la revendication 1, dans lequel le copolymère séquencé consiste en un polyester-polyéther et le polymère consiste en une résine phénoxy.

5. Procédé suivant l'une quelconque des revendications précédentes, dans lequel la température de transition prédéterminée est égale à la température de l'organisme et la première configuration consiste en un cathéter.

6. Procédé suivant l'une quelconque des revendications 1 à 4, dans lequel la température de transition prédéterminée est la température de l'organisme et l'article est un dispositif à libération de médicament.

7. Procédé suivant l'une quelconque des revendications 1 à 4, dans lequel la température de transition prédéterminée est la température de l'organisme et l'article est un indicateur de température.

8. Procédé suivant l'une quelconque des revendications précédentes, dans lequel la température de transition vitreuse prédéterminée est comprise dans l'intervalle d'une valeur supérieure de 10°C à la température de l'organisme à 62°C.

9. Procédé suivant la revendication 1, dans lequel l'article est un jouet.